(19) Europäisches Patentamt
European Patent Office
Office européen des brevets

(11) Publication number: **0 609 899 A1**

# (12) EUROPEAN PATENT APPLICATION

(21) Application number: **94101742.8**

(22) Date of filing: **04.02.94**

(51) Int. Cl.[5]: **C07D 307/62**, C07D 307/66, A61K 31/34

(30) Priority: **05.02.93 HR 13193**

(43) Date of publication of application: **10.08.94 Bulletin 94/32**

(84) Designated Contracting States: **AT BE CH DE FR GB IT LI NL SE**

(71) Applicant: **PLIVA FARMACEUTSKA KEMIJSKA PREHRAMBENA I KOZMETICKA INDUSTRIJA DIONICKO DRUSTVO ZAGREB**
**Vukovarska av. 49**
**HR-41000 Zagreb (HR)**

(72) Inventor: **Suskovic, Bozidar**
**Tuskanac 57c**
**HR-41000 Zagreb (HR)**
Inventor: **Dr. Vela, Vanja**
**Gajnice 15**
**HR-41000 Zagreb (HR)**
Inventor: **Buncic, Mira**
**Rudeska cesta 162**
**HR-41000 Zagreb (HR)**

(74) Representative: **von Füner, Alexander, Dr. et al**
**Patentanwälte v. Füner, Ebbinghaus, Finck**
**Mariahilfplatz 2 & 3**
**D-81541 München (DE)**

## (54) Derivatives of amino-ascorbic acid,the processes for their preparation and use.

(57) The invention relates to the process for the preparation of new derivatives of amino-ascorbic acid of formula I,

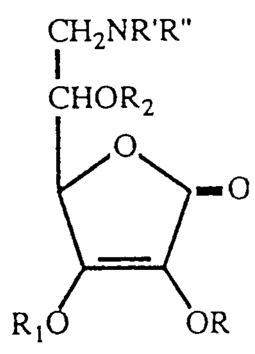

I

their acid and alkali salts, processes for preparation and action.

According to this invention derivatives of amino-ascorbic acid are prepared by three types of reactions: 1. reductive alkylation of amino-ascorbic acid; 2. alkylation of primary and secondary amines with halogen-ascorbic acid and 3. acylation of amino-ascorbic acid.

EP 0 609 899 A1

In continuation of our works on the preparation of the derivatives of ascorbic acid in the position $C_6$ /Yug. pat. Application P-1852/88, Croat. Chim. Acta 62 (3), 537 - 544 (1989)/, and the studies of the relations between the structure /Acta Cryst. C45, 269 - 273 (1989), Croat. Chem. Acta 64 (3) (1991)/ and biological activity (Re. Exp. Med. 190, 443 - 449 (1990)/, prepared were new derivatives of 6-deoxy-6-amino-ascorbic acid.

The present invention relates to new derivatives of amino-ascorbic acid of the general formula (**I**)

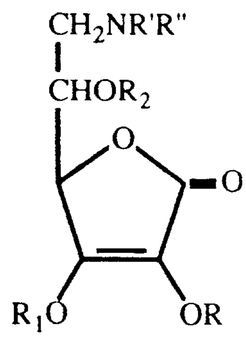

I

wherein R' = H, $C_1$ - $C_{18}$ alkyl atoms, $C_6$ cycloalkyl atoms, $C_6$ - $C_{12}$ aryl atoms, heteroyl atoms, which represents a heterocyclic compound containing in the ring oxygen, sulfur or nitrogen as heteroatom, R'' = $C_1$ - $C_{18}$ alkyl atoms, $C_6$ cycloalkyl atoms, $C_6$ - $C_{12}$ aryl atoms, heteroyl atoms, which represents a heterocyclic compound containing in the ring oxygen, sulfur or nitrogen as heteroatom, R'R'' = $CH_2R_3$ wherein $R_3$ = $C_1$ - $C_{18}$ alkyl atoms, $C_6$ cycloalkyl atoms, $C_6$ - $C_{12}$ aryl atoms, heteroylatoms, representing a heterocyclic compound containing in the ring oxygen, sulfur or nitrogen as heteroatom, i. e. R' = H, R'' = acyl, i2 e. R'' = COR''' wherein R''' = H, $C_1$ - $C_{18}$ alkyl atoms, $C_6$ cycloalkyl atoms, $C_6$ - $C_{12}$ aryl atoms, heteroyl atoms, which represents a heterocyclic compound containing in the ring oxygen, sulfur or nitrogen as heteroatom, R, $R_1$ and $R_2$ = H or a protecting group which is easily removed in neutral media, such as is benzyl. The new compounds which contain an (un)substituted amino- group form salts with acids, whereas when R = $R_1$ = $R_2$ = H salts are formed with bases. Unprotected compounds of the formula (**I**) in the neutral media occur in the form of zwitter-ion.

New derivatives of ascorbic acid (**I**) which are object of present invention, can be prepared in several ways from the compounds of the general formula (**II**)

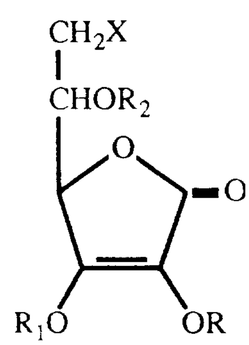

II

wherein X = halogen (Cl, Br, J) or amino ($NH_2$). When X = halogen, R = $R_1$ = benzyl, $R_2$ = H, i. e. R = $R_1$ = $R_2$ = benzyl, and when X=amino, R = $R_1$ = $R_2$ = H. New compounds are produced by several types of reactions, illustrated by the following schemes:

1. Reductive alkylation

Reductive alkylation is the treatment of aldehyde or ketone with ammonia or primary, i. e. secondary amines in the presence of hydrogen and a catalyst for hydrogenation. Instead of hydrogen and a catalyst, can be used other reductants, such as borohydrides and formic acid. In the present invention reductive alkylation is carried out with amino-ascorbic acid (II, X = $NH_2$, R = $R_1$ = $R_2$ = H) with various aldehydes of $R_3CHO$, which is illustrated by the scheme 1:

Scheme 1. Reductive alkylation of amino-ascorbic acid

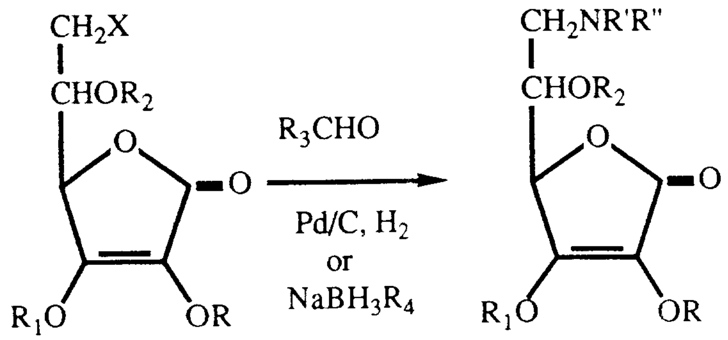


II

X = $NH_2$
$R=R_1=R_2=H$

I

$R'R''=(CH_2R_3)_2$
$R=R_1=R_2=H$

Reductive alkylation is carried out in water as solvent at the temperatures of 20 - 25 °C. Used reductant is hydrogen with catalyst 5% or 10% Pd/C, or sodium borohydride wherein $R_4$ = H or CN. In case catalytic reduction is applied reaction time is 1 - 2 hours, whereas with borohydrides the reaction takes 5 - 10 hours. The mechanism of reductive alkylation illustrated by the scheme 1a

Scheme 1a.

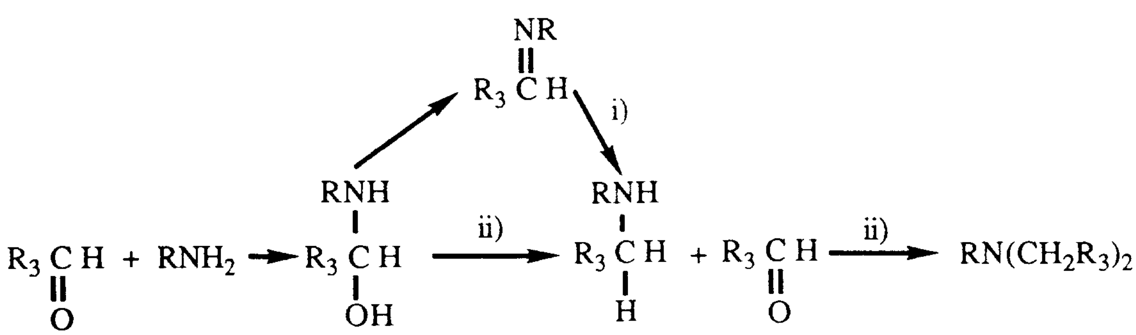


allows obtaining of mono- and disubstituted derivatives of amino-ascorbic acid. In our inovation however detected and isolated have been only disubstituted derivatives. This suggests that the reaction of alkylation in the second phase occurs very rapidly.

2. Alkylation reaction

Mono- and disubstituted derivatives of amino-ascorbic acid are prepared by alkylation of corresponding primary and secondary amines, according to the reaction scheme 2.

Scheme 2. Alkylation reaction

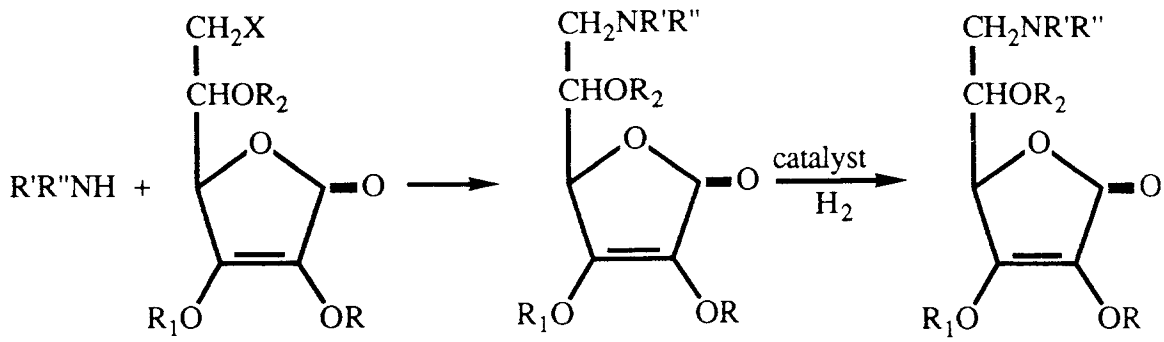


II — X = Hal. R=H, $R_1$=$R_2$=benzyl or R=$R_1$=$R_2$=benzyl

I — R=H, $R_1$=$R_2$=benzyl or R=$R_1$=$R_2$=benzyl

I — R=$R_1$=$R_2$=H

In alkylation reactions are used primary and secondary amines of the general formula NHR'R" wherein R' and R" are as already described for the compounds of the general formula (**I**). In this reaction the alkylating reagent is halogen-ascorbic acid of the general formula (**II**). Because halogen-ascorbic acid has three more hydroxy groups of which hydroxy groups of enediol system in the positions $C_2$ and $C_3$ are very reactive in the alkaline medium, it is necessary to protect the molecule adequately prior to the reaction. In other words it is necessary to select protecting groups which are removed in neutral media, such as is benzyl group. The protecting reaction is carried out according to the scheme 2a.

Scheme 2a. Protection of hydroxyl groups of halogen-ascorbic acid

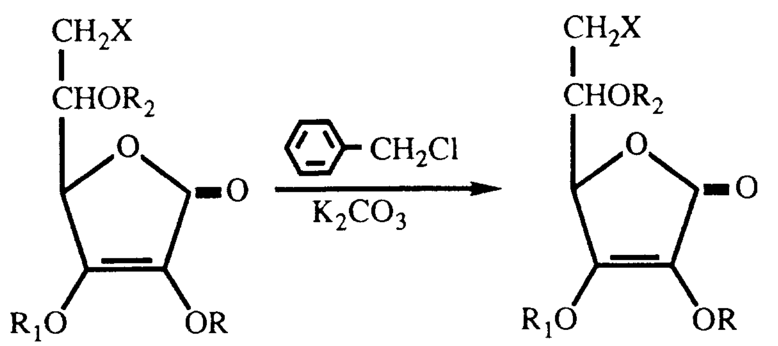


II — X = Hal. R=$R_1$=$R_2$=H

II — X = Hal. R=$R_1$=$R_2$=benzyl

Product of the reaction depends upon molar proportion of the reactants. If for one mole of halogen-ascorbic acid two moles of the protecting reagents are used, produced is the compound of the general formula (**II**) wherein R = $R_1$ = benzyl, $R_2$ = H, and if three moles or larger molar excess of this reagent is used,

obtained is a fully protected compound. Deprotection of the protecting groups is carried out in the neutral media by catalytic reduction, as is illustrated by the scheme 2. Also the reaction can be carried out without isolation of an intermediate by the addition of a catalyst to the reaction mixture after alkylation is finished, and thereafter by deprotection (one-pot-reaction) under hydrogen atmosphere, wherein it has been noted that in the fully protected compound the protecting group $R_2$ is not easy to deprotect, and therefore in such types of reactions mostly used are the compounds of the general formula (**II**) wherein $R = R_1$ = benzyl, $R_2$ = H.

3. Acylaton reaction

Acylating reaction of amino- group is carried out by the reagents commonly mentioned in chemical literature, such as are chlorides or anhydrides of acids or active esters of acids i. e. by the reagents of the general formula:

$R_5COR_6$

wherein $R_5$ = H, alkyl, cycloalkyl, aryl or heteroil having the meaning as described for the formula (**I**), and $R_6$ = Cl, or $OCOR_7$ wherein $R_7$ = alkyl, or $OR_8$ wherein $R_8$ is the group known in chemistry as the active ester group, such as are succinyl or benzotriazolyl.

Acylation reaction is illustrated by the scheme 3.

Scheme 3. Acylation of amino- group

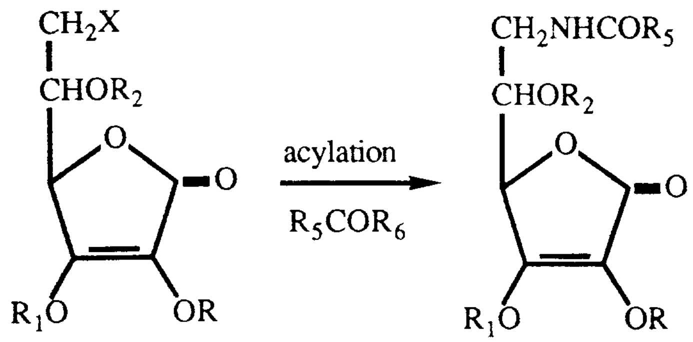


II

$X=NH_2$
$R=R_1=R_2=H$

I

$R=R_1=R_2=H$

If the reaction of amine acylation is carried out with formic acid ($R_5$ = $R_6$ = OH) or with a mixed anhydride of formic and acetic acids ($R_5$ = H, $R_6$ = $OCOR_7$, $R_7$ = $CH_3$), resulting is N-formyl derivative. It is a known fact that under the reductive conditions N-formyl derivatives can be converted to the corresponding monomethyl amino-derivatives. Amino-ascorbic acid however has shown to produce acyl derivatives which are very stable under reductive conditions. For example formyl amino-ascorbic acid is not reduced at the pressure of 80 bar and at 80 °C and acyl derivatives of amino-ascorbic acid are far more stabile at high temperatures than ascorbic acid. Under mentioned conditions maintained over 8 hours chromatography cannot record any disintegration of the molecule. As the derivatives of acylamino-ascorbic acid retain their reducing property they can be used as antioxidants at the temperatures exceeding that for ascorbic acid.

New derivatives of amino-ascorbic acid demonstrate anticarcinogenic properties which have been tested on the following lines of tumor cells: HeLa (carcinoma of the portio vaginalis cervicis); Hep 2 (carcinoma of the larynx); MiaPaCa 2 (carcinoma of the pancreas) and K562 (erythroleukemia), all human cell lines and W138 (normal human fibroblasts) which are used as the control.

Cell lines of human tumors and human fibroblasts are grown on the liquid DMEM media (Dulbecco's modified Eagle's medium) with the addition of 10% of the calf fetus serum (FCS), 2mM glutamine, 100 U/ml

penicillin and 100 μg/ml streptomycin in the humid atmosphere with 5% $CO_2$ at 37°C. The cells are inoculated in the concentration of $10^4$ cells/ml.

The samples are dissolved in DMEM without the addition of serum, and 0.1N NaOH is added till pH 7.4. Used concentrations are $10^{-3}$M, $2x10^{-3}$M and $3x10^{-3}$M. The results illustrated in Fig. 1 represent mean values of four parallel samples ± standard deviation (SD). The results are illustrated by the Fig. 1.

Fig 1

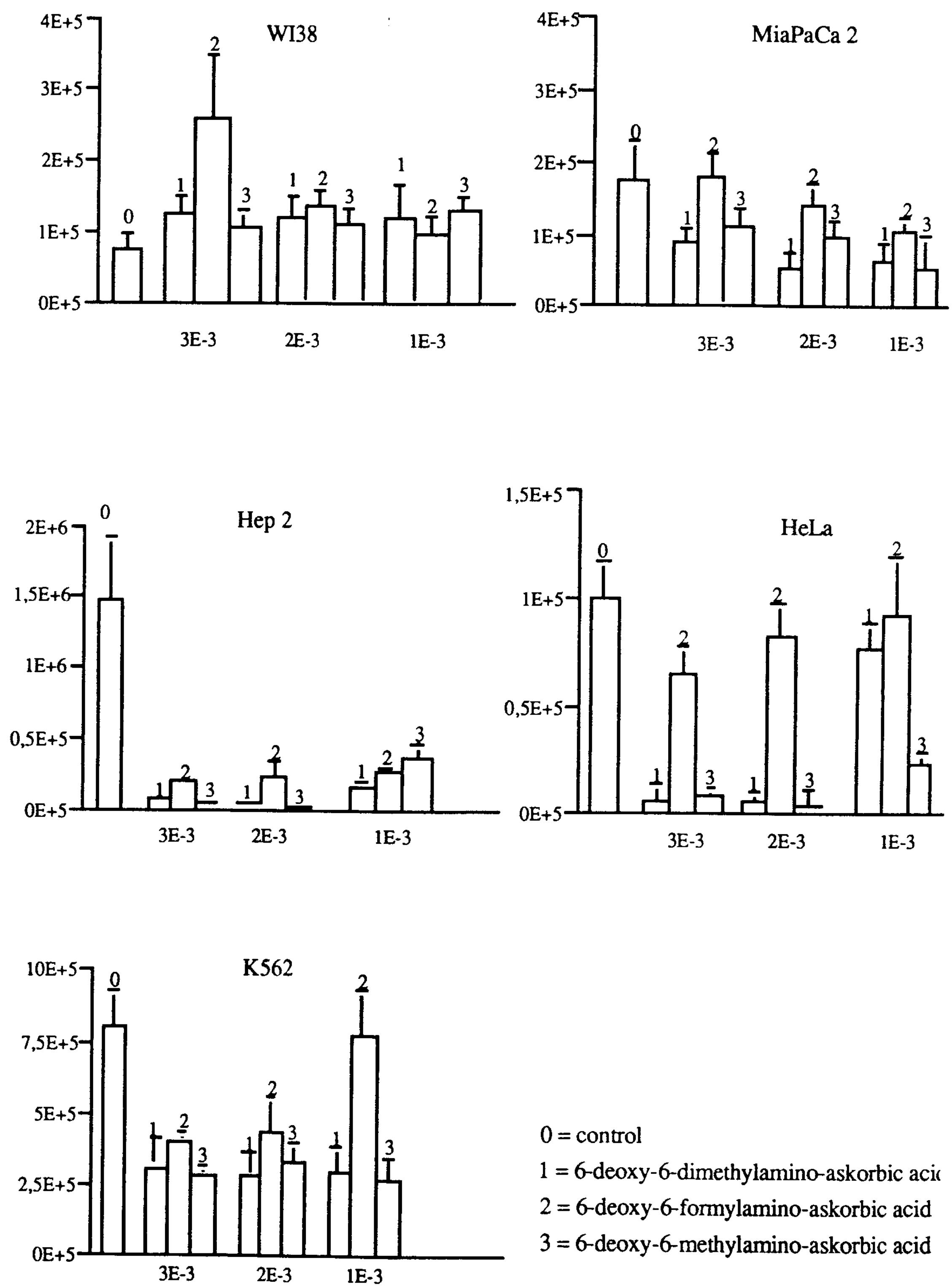

As can be noted from the Fig. 1 the growth of normal fibroblast W138 cells is not affected by the tested derivatives which do inhibit the growth of tumor cells, especially Hep 2.

Preparation of new derivatives of amino-ascorbic acid is illustrated by the following examples, which do not limit the inovation at any point.

**Examples of reductive alkylation**

**Example 1**

**N,N'-dimethylamino-ascorbic acid**
**(I, R' = R''= $CH_3$, R = $R_1$ = $R_2$ = H)**

Process a): reduction with the aid of the Pd/C catalyst.

To the solution of amino-ascorbic acid (II, X = $NH_2$, R = $R_1$ = $R_2$ = H) in water (100 ml) 35% water solution of formaldehyde (2.4 ml) and catalyst 10% Pd/C (0.48 g) are added, whereafter stirred at hydrogen pressure of 0.8 bar in the bottle according to Parr for 1 hour. Catalyst is removed by filtration, and water solution evaporated to the volume of approximately 2 - 3 ml . The concentrate is purified by column chromatography on silica gel (Kieselgel 60 0.063 - 0.2 mm art. 7734), by elution with the solvent methanol-water = 7:3. Fractions which contain the product (tlc, silica gel 60 $F_{254}$ Merck, detection spraying with phosphoromolybdenic acid) are combined, and thereafter evaporated to dryness to yield 0.3 g of the product, m. p. 207 - 210 °C; = $[a]_D^{20}$ = +46.5° (c = 0.1, $H_2O$).
IR (KBr) ($cm^{-1}$): 3400, 2900, 2800, 2350, 1750, 1650, 1620, 1480, 1430, 1100, 1050.
$^1H$ NMR ppm (d) ($D_2O$): 2.83 (6H, s), 3.22 - 3.37 (2H, m) 4.2 - 4.25 (1H, m), 4.28 - 4.29) (1H, d, J = 2.17 Hz).
$^1H$ NMR ppm (d) ($CF_3COOD$): 3.2 - 3.23 (6H, d, J = 8.79 Hz), 3.58 - 3.83 (2H, m), 4.79 - 4.83 (1H, m), 5.02 (1H, s).
$^{13}C$ NMR ppm (d) ($CF_3COOD$ + $D_2O$): 40.97, 44.19, 58.47, 62.98, 75.43, 152.43, 161.23.
EI-MS m/s: 203 ($M^+$), 58, 82, 88, 112, 130.

**Example 2**

Process b): reduction with borohydride

To the solution of amino-ascorbic acid (0.5 g) in water (50 ml) 35% water solution of formaldehyde (1 ml), sodium cyanoborohydride ($NaBH_3CN$, 0.537 g) and cold acetic acid (0.3 g) are added, thereafter stirred in nitrogen atmosphere for 5 hours at 20 - 25 °C. The reaction mixture is then concentrated to the volume of approximately 1 - 2 ml, purified by column chromatography on silica gel, and thereafter eluted with solvent methanol-water = 7:3. Fractions which contain the product (tlc) are combined, and thereafter evaporated to dryness to yield 0.28 g of the product identical with that from Example 1.

**Example 3**

**N,N'-di-n-propylamino-ascorbic acid**
**(I, R'= R''= $C_3H_7$, R = $R_1$ = $R_2$ = H)**

To the solution of amino-ascorbic acid (0.32 g) in water (15 ml) n-propanol ($C_3H_6O$, 0.11 g), sodium cyanoborohydride ($NaBH_3CN$, 0.32 g), and thereafter cold acetic acid (0.2 ml) are added, whereafter the reaction mixture is stirred in nitrogen atmosphere for 3 hours at 20 - 25 °C. The reaction mixture is concentrated to the volume of approximately 1 ml, and purified by column chromatography on silica gel, by elution with solvent ethanol-water = 1:1. Fractions which contain the product (tlc, silica gel 60 $F_{254}$, EtOH:$H_2O$ = 9:1) are evaporated to dryness to yield 0.2 g of the product.
$^1H$ NMR ppm (d) ($D_2O$): 0.9 - 0.94 (6H, t) 1.69 - 1.7 (4H, m), 3.11 - 3.2 (4H, t), 3.37 - 3.38 (2H, m), 3.4 - 3.45 (1H, m), 4.37 - 4.38 (1H, d).

**Example 4**

**N,N'-dibenzylamino-ascorbic acid**
**(I, R' = R'' = $CH_2C_6H_5$, R = $R_1$ = $R_2$ = H)**

To the solution of amino-ascorbic acid (0.36 g) in water (20 ml) benzaldehyde (0.228 g), sodium cyanoborohydride (0.382 g) and cold acetic acid (0.2 ml) are added, thereafter stirred in nitrogen atmosphere for 3 hours at 20 - 25°C. The reaction mixture is concentrated to the volume of approximately 1 ml, and purified by column chromatography on silica gel (Kieselgel 60 0.063 - 0.2 mm art. 7734), by elution with solvent ethanol-water = 8:2. Fractions which contain the product (tlc) are combined, and thereafter evaporated to dryness to yield 0.25 g of the product.
$^1$H NMR ppm (d) ($D_2O$): 3.92 (2H, s), 4.31 - 4.35 (1H, m), 4.59 - 4.61 (1H, d, J = 2.15 Hz), 4.83 (4H, s), 7.59 - 7.69 (10H, m).

**Example 5**

**N,N'-difurfurylamino-ascorbic acid**
**(I, R' = R'' = $CH_2C_4H_3O$, R = $R_1$ = $R_2$ = H)**

To the solution of amino-ascorbic acid (0.52 g) in water (26 ml) furfural (1.45 g), sodium cyanoborohydride (0.565 g) and cold acetic acid (0.5 ml) are added, whereafter the reaction mixture is mixed in nitrogen atmosphere for 5 hours at 20 - 25°C. The mixture is concentrated at reduced pressure to the volume of 2 ml, and purified by column chromatography on silica gel, by elution with solvent ethanol-water = 8:2. Fractions which contain the product (tlc) are combined, and thereafter evaporated to dryness to yield 0.7 g of the product.
mp = 220°C (decomposition): = $[a]_D^{20}$ = +22° (c = 0.58, $H_2O$).
IR (KBr) ($cm^{-1}$): 3550, 3500, 3450, 2350, 2200, 1630, 1380, 1100, 750.

**Examples of alkylating reactions**

**Example 6**

**N-methylamino-ascorbic acid**
**(I, R' = H, R'' = $CH_3$, R = $R_1$ = $R_2$ = H)**

To the solution of 2,3-dibenzyl-6-bromo-6-deoxy-ascorbic acid (II, X = Br, R = $R_1$ = Bn, $R_2$ = H) (0.5 g) in methanol (10 ml), 20% methanol solution of methylamine (10 ml) is added and stirred for 1 hour, whereafter it is allowed to stand overnight at 20°C. The reaction mixture is concentrated at reduced pressure to dryness. To the residue methanol (15 ml) and catalyst 10% Pd/C (0.015 g) are added and stirred at hydrogen pressure of 2 bar in the bottle according to Parr for 3 hours. Catalyst is separated by filtration, thereafter the solution concentrated at reduced pressure to the volume of 2 ml, and then purified by column chromatography on silica gel, by elution with solvent methanol-water 8:2. Fractions which contain the product (tlc) are combined, and thereafter evaporated to dryness to yield 0.05 g of the product.
mp = 155 - 160°C; IR (KBr) ($cm^{-1}$): 3400, 2900, 2810, 2380, 1740, 1600, 1480, 1380, 1150, 1110, 1050.
$^1$H NMR ppm (d) ($D_2O$): 2.72 (3H, s), 3.21 - 3.29 (2H, m), 3.73 - 4.29 (1H, m), 4.37 - 4.40 (1H, d, J = 2.2 Hz).

**Example 7**

**N,N'-dimethylamino-ascorbic acid**
**(I, R' = R'' = $CH_3$, R = $R_1$ = $R_2$ = H)**

To the solution of 2,3-dibenzyl-6-bromo-6-deoxy-ascorbic acid (II, X = Br, R = $R_1$ = Bn, $R_2$ = H) (0.27 g) in methanol (5 ml), 20% methanol solution of dimethylamine (5 ml) is added, stirred for 1 hour, and thereafter allowed to stand for 24 hours at 20 - 25°C. The reaction mixture is concentrated at reduced pressure to dryness. The residue is dissolved in methanol (10 ml) wherein 10% Pd/C as a catalyst (0.01 g) is added and stirred at hydrogen pressure of 2 bar in the bottle according to Parr for 3 hours. Catalyst is separated by filtration, and methanol concentrated at reduced pressure to the volume of 2 ml. Thereafter it

is purified by column chromatography on silica gel, by elution with solvent methanol-water 8:2. Fractions which contain the product (talc) are combined, and thereafter evaporated to dryness to yield 0.03 g of the product which is identical with that from the Example 1.

**Protection of hydroxy groups of halogen-ascorbic acid**

**Example 8**

**2,3,5-tribenzyl-6-bromo-6-deoxy-ascorbic acid**
**(II, X = Br, R = $R_1$ = $R_2$ = Bn)**

To the solution of 6-bromo-6-deoxy-ascorbic acid (II, X = Br, R = $R_1$ = $R_2$ = H) (1 g) in dimethylformamide (15 ml), potassium carbonate (0.91 g) and benzylchloride (1.64 g) are added, and thereafter stirred in hydrogen atmosphere for 2 hours at 60 °C. The reaction mixture is concentrated at reduced pressure to dryness, whereafter to the residue chloroform (15 ml) and water (5 ml) are added. Organic layer is separated and washed twice with water (5 ml), and thereafter dried over sodium sulfate sicc. Chloroform solution is concentrated at reduced pressure to the volume of 2 ml, whereafter it is purified by column chromatography on silica gel, by elution with solvent chloroform-ethanol 32:1. Fractions which contain the product are combined, and thereafter evaporated to dryness to yield 0.2 g of the product.
IR $CHCl_3$ ($cm^{-1}$): 3400, 3090, 3060, 3040, 2950, 1760, 1670, 1500, 1460, 1320, 1210, 1150, 1050.
$^1H$ NMR ppm (d) ($CDCl_3$): 3.51 - 3.54 (2H, m), 4.03 - 4.07 (1H, m), 4.91 - 4.92 (1H, d, J = 2.14 Hz), 5.08 (2H, s), 5.09 (2H, s), 5.16 - 5.19 (2H, d), 7.20 - 7.37 (15 H, m).

**Example 9**

**Process for the preparation of 2,3-dibenzyl-6-bromo-6-deoxy-ascrobic acid**
**(II, X = Br, R = $R_1$ = Bn, $R_2$ = H)**

To the solution of 6-bromo-6-deoxy-ascorbic acid (II, X = Br, R = $R_1$ = $R_2$ = H) (2 g) in dimethylformamide (30 ml) potassium carbonate (1.2 g) and benzyl chloride (2.2 g) are added, whereafter stirred in nitrogen atmosphere for 2 hours at 60 °C. Reaction mixture is concentrated at reduced pressure to oily residue. To the residue chloroform (30 ml) and water (10 ml) are added. Organic layer is separated and washed with water (2 x 10 ml), and then dried over sodium sulfate sicc. The solution of chloroform is concentrated to the volume of 2 ml, whereafter it is purified by column chromatography on silica gel, by elution with solvent methylene chloride-ethanol (80:1). Fractions which contain the product (tlc) are combined, and thereafter evaporated to oily precipitate which has all physico-chemical properties described in the literature[1].

**Acylation reaction**

**Example 10**

**Formylamino-ascorbic acid**
**(I, R' = H, R'' = CHO, R = $R_1$ = $R_2$ = H)**

Anhydride of acetic and formic acid (3.7 ml) is prepared by stirring acetic acid anhydride (2.5 ml) and formic acid) (1.2 ml) for 2 hours at 50 - 60 °C. Thereafter it is cooled to 0 °C. Amino-ascorbic acid (I, R' = R'' = H, R = $R_1$ = $R_2$ = H, 0.5 g) is dissolved and stirred for 4.5 hours at 20 - 24 °C. The reation mixture is concentrated at reduced pressure, evaporated after the addition of water (5 ml), and then dissolved in 2 ml water. Thereafter it is purified by column chromatography on silica gel. Next, it is eluted with solvent methanol-acetonitrile 8:2. Fractions which contain the product (tlc) are combined and concentrated to dryness at lower pressure to yield 0.26 of the product.
mp = 200 °C (decompos.) IR (KBr) ($cm^{-1}$): 3450, 2900, 2350, 1740, 1670, 1600, 1390, 1250, 1150, 1110, 1050.
$^1H$ NMR ppm (d) ($D_2O$): 3.31 - 3.54 (2H, m), 3.97 - 4.02 (1H, m), 4.42 (1H, d, J = 2.3 Hz), 8.03 (1H, s).
$^{13}C$ NMR ppm (d) ($CF_3COOD$ + $D_2O$): 43.2, 66.0, 78.2, 119.3, 153.8, 166.9, 174.0
EI-MS m/s: 55, 56, 57, 59, 62, 69, 70, 73, 84 ,85, 86, 87, 103, 113.

References Cited:

1. Von F. Dallacker and J. Sanders Chem.-Zeitung. **109** (1985) 277 - 280.

The solution of amino ascorbic acid (I, R',R" = H, R = $R_1$ = $R_2$ = H, 0.5 g) in the mixed anhydride of formic and acetic acid (3.7 ml) whic is prepared by stirring acetic acid anhydride (2,5 ml) and formic acid (1.2 ml) for 2 hours at 50-60 ° C, followed by cooling at 0 ° C, is stirred for 4,5 hours at 20-24 ° C.

## Claims

1. Derivatives of amino-ascorbic acid of the general formula (I):

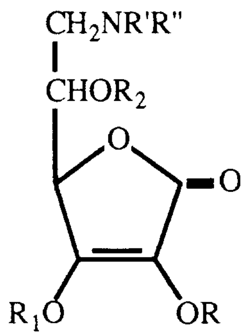

I

wherein R' = H, $C_1$ - $C_{18}$ alkyl atoms, $C_6$ cycloalkyl atoms, $C_6$ - $C_{12}$ aryl atoms, heteroyl atoms, which represents a heterocyclic compound containing in the ring oxygen, sulfur or nitrogen as heteroatom, R" = $C_1$ - $C_{18}$ alkyl atoms, $C_6$ cycloalkyl atoms, $C_6$ - $C_{12}$ aryl atoms, heteroyl atoms, which represents a heterocyclic compound containing in the ring oxygen, sulfur or nitrogen as heteroatom, R'R" = $CH_2R_3$ wherein $R_3$ = $C_1$ - $C_{18}$ alkyl atoms, $C_6$ cycloalkyl atoms, $C_6$ - $C_{12}$ aryl atoms, heteroyl atoms, representing a heterocyclic compound containing in the ring oxygen, sulfur or nitrogen as heteroatom, i. e. R' = H, R" = COR"' wherein R"' = H, $C_1$ -$C_{18}$ alkyl atoms, $C_6$ cycloalkyl atoms, $C_6$ - $C_{12}$ aryl atoms, heteroyl atoms, which represens a heterocyclic compound containing in the ring oxygen, sulfur or nitrogen as heteroatom and R, $R_1$ and $R_2$ = H or benzyl and their salts with acids and alkali.

2. Derivative of amino-ascorbic acid of the formula (**I**) according to claim 1 is characterized by R' = R" = methyl and R = $R_1$ = $R_2$ = H.

3. Derivative of amino-ascorbic acid of the formula (**I**) according to claim 1 is characterized by R' = R" = propyl and R = $R_1$ = $R_2$ = H.

4. Derivative of amino-ascorbic acid of the formula (**I**) according to claim 1 is characterized by R' = R" = benzyl and R = $R_1$ = $R_2$ = H.

5. Derivative of amino-ascorbic acid of the formula (**I**) according to claim 1 is characterized by R' = R" = furfuryl and R = $R_1$ = $R_2$ = H.

6. Derivative of amino-ascorbic acid of the formula (**I**) is characterized by R' = H, R" = methyl and R = $R_1$ = $R_2$ = H.

7. Derivative of amino-ascorbic acid of the formula (**I**) according to claim 1 is characterized by R' = H, R" = formyl and R = $R_1$ = $R_2$ = H.

8. Derivative of amino-ascorbic acid of the formula (**I**) according to claim 1 is characterized by R' = R" = methyl, R = $R_1$ = benzyl and $R_2$ = H.

**9.** Derivative of amino-ascorbic acid of the formula (**I**) according to claim 1 is characterized by R' = H, R'' = methyl and R = $R_1$ = benzyl, $R_2$ = H.

**10.** Derivatives of halogen-ascorbic acid of the general formula (**II**):

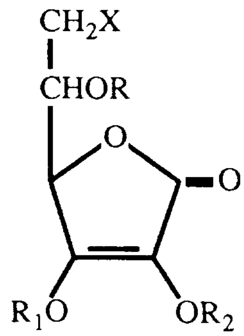

II

wherein X = chlorine, bromine or iodine and R = $R_1$ = $R_2$ = benzyl.

**11.** Derivative of halogen-ascorbic acid of the general formula (**II**) is characterized by X = bromine, R = $R_1$ = $R_2$ = benzyl.

**12.** Process for the preparation of the compounds of the general formula (**I**) is characterized by being obtained from the compounds of the general formula (**II**) wherein X = $NH_2$ and R = $R_1$ = $R_2$ = H, by the reductive alkylation of the compounds of the general formula:

$R_3CHO$

wherein $R_3$ = $C_1$ - $C_{18}$ alkyl atoms, $C_6$ - $C_{12}$ aryl atoms or heteroil, which represents a heterocyclic compound containing oxygen, nitrogen or sulfur as heteroatom in the atmosphere of hydrogen in the presence of metal catalysts or in the presence of borohydrides of the general formula:

$NaBH_3R_4$

wherein $R_4$ = H or CN as reductants.

**13.** Process for the preparation of the compounds of the general formula (**I**) is characterized by the compounds of the general formula:

R'R''NH

wherein R' and R'' have the meaning as described for the compounds of the formula (**I**), being alkylated with the compounds of the general formula (**II**) wherein X = halogen, R = $R_1$ = benzyl, $R_2$ = H or R = $R_1$ = $R_2$ = benzyl.

**14.** Process for the preparation of the compounds of the general formula (**I**) wherein R' = H, R'' = $COR_5$, wherein $R_5$ = H, $C_1$ - $C_{18}$ alkyl atoms, aryl or heteroyl, and R = $R_1$ = $R_2$ = H or benzyl, i. e. R = $R_1$ = benzyl, $R_2$ = H, is characterized by the compounds of the general formula (**II**) wherein X = $NH_2$, R = $R_1$ = $R_2$ = H or benzyl, i. e. R = $R_1$ = benzyl, $R_2$ = H, being acylated with the compounds of the general formula:

$R_5COR_6$

wherein $R_6$ = chlorine, $OCOR_7$ or $OR_8$, in which $R_7$ = alkyl and $R_8$ = succinyl or hydroxybenzotriazolyl.

**15.** Process for the preparation of the derivatives of the general formula (**II**) wherein X = bromine and R = $R_1$ = benzyl, $R_2$ = H.

**16.** Derivatives of amino-ascorbic acid according to the claim 1 are characterized by being used as antioxidants.

**17.** Derivatives of amino-ascorbic acid according to the claim 1 are characterized by being used as anticancerogenic agents.

**18.** Derivatives of amino-ascorbic acid according to the claim 1 are characterized by being used as intermediates for obtaining of new derivatives of ascorbic acid.

European Patent Office

# EUROPEAN SEARCH REPORT

Application Number
EP 94 10 1742

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 446 539 (MERRELL DOW PHARMACEUTICALS INC.) 18 September 1991<br>* abstract; claims; examples 2,4,5 * | 1-11,13, 15-18 | C07D307/62<br>C07D307/66<br>A61K31/34 |
| X | DE-A-26 16 351 (HOFFMANN-LA ROCHE & CO AG) 11 November 1976 | 10,15 | |
| X | EP-A-0 096 930 (PROCTER & GAMBLE CO.)<br>RN's [62983-46-4, 89130-94-9]<br>* abstract; examples * | 10 | |
| X | EP-A-0 096 933 (PROCTER & GAMBLE CO.)<br>RN's [62983-43-1 and 89130-95-0]<br>* abstract; examples * | 10 | |
| X | CHEMICAL ABSTRACTS, vol. 117, no. 7, 17 August 1992, Columbus, Ohio, US;<br>abstract no. 65658z,<br>F. YAMAMOTO ET AL. 'Positron labeled antioxidants: synthesis and tissue biodistribution of 6-deoxy-6[18F]fluoro-L-ascorbic acid'<br>page 386 ;column 2 ;<br>* abstract *<br>& APPL. RADIAT. ISOT.<br>vol. 43, no. 5 , 1992<br>pages 633 - 639 | 10,15 | |
| | | | TECHNICAL FIELDS SEARCHED (Int.Cl.5)<br>C07D |
| D,X | CHEMIKER-ZEITUNG<br>vol. 109, no. 9 , 1985 , HEIDELBERG DE<br>pages 277 - 280<br>F. DALLACKER ET AL. 'Derivatives of L-ascorbic acid. 2. Preparation of deoxy-L-ascorbic acid.'<br>* the whole document *<br>-/-- | 10,15 | |

The present search report has been drawn up for all claims

| Place of search | Date of completion of the search | Examiner |
|---|---|---|
| THE HAGUE | 19 April 1994 | Paisdor, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 10 1742

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X | CHEMICAL ABSTRACTS, vol. 113, no. 1, 2 July 1990, Columbus, Ohio, US; abstract no. 6058y, B. SUSKOVIC 'Preparation of 6-amino-6-deoxy derivatives of ascorbic and iso-ascorbic acid' page 588 ;column 2 ; * abstract * & CROAT. CHEM. ACTA vol. 62, no. 3 , 1989 pages 537 - 544 --- | 10,15 | |
| A | JERRY MARCH 'Advanced Organic Chemistry, Reactions Mechanisms, and Structure; Third Edition' 1985 , JOHN WILEY & SONS, INC. , NEW YORK US * page 798 - page 800, paragraph 6-15 * ----- | 12 | |
| | | | TECHNICAL FIELDS SEARCHED (Int.Cl.5) |

The present search report has been drawn up for all claims

| Place of search | Date of completion of the search | Examiner |
|---|---|---|
| THE HAGUE | 19 April 1994 | Paisdor, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document